# EUROPEAN PATENT APPLICATION

(11) **EP 2 514 416 A1**
(43) Date of publication of application: **24.10.2012**
(21) Application number: 10837662.5
(22) Date of filing: 16.12.2010
(51) Int. Cl.: A61K 9/70, A61K 31/13, A61K 31/27, A61K 31/445, A61K 31/473, A61K 31/55, A61K 45/00, A61K 47/10, A61K 47/14, A61K 47/32, A61P 25/28

(54) **TRANSDERMALLY ABSORBED PREPARATION OF ANTI-DEMENTIA DRUG**

(30) Priority: 16.12.2009 JP 2009285472
(71) Applicant: Goto, Takeshi, Ushiku-shi, Ibaraki 300-1206 (JP)
(72) Inventor: ITO Takeshi, Fukuoka-shi Fukuoka 819-0043 (JP)
(74) Representative: Tetaz, Franck Claude Edouard
(86) International application number: PCT/JP2010/072668
(87) International publication number: WO 2011/074637

(57) **Abstract**

The present invention relates to a percutaneous absorption preparation comprising an anti-dementia drug, which is lower skin irritation. More specifically, the present invention relates to a percutaneous absorption preparation comprising a drug-containing layer comprising an anti-dementia drug, a polymer compound having an amino group, a polyhydric alcohol fatty acid ester, a polyhydric alcohol, a polyvalent carboxylate ester, and a styrenic polymer compound, wherein the content of the anti-dementia drug is 0.5-20 mass% of the drug-containing layer.

## Description

### [Cross-Reference to Related Application]

This application claims priority to Japanese Patent Application No. 2009-285472, filed on December 16, 2009, the entire disclosure of which is incorporated herein by reference.

### [Technical Field]

The present invention relates to a percutaneous absorption preparation comprising an anti-dementia drug, which is lower skin irritation.

### [Background Art]

In recent years, with increase in the number of elderly persons, the number of patients with dementia such as Alzheimer has also been increased and care for the patients and the like have become social problems. On the other hand, the development of anti-dementia drugs has also rapidly been pursued and, for example, donepezil hydrochloride has widely been used as a therapeutic agent for Alzheimer's disease, having an acetylcholinesterase inhibitory action. These anti-dementia drugs have been often orally administered via tablets and the like. As methods for administering drugs to patients, oral administration of tablets, capsules, syrups, granules, and the like as well as injection administration, rectal administration, and the like have been known and appropriately selected depending on the diseases of the patients and the features of the drugs.

However, a patient with an advanced symptom of dementia often gets into difficulty in taking an anti-dementia drug. Accordingly, the transdermal administration of the anti-dementia drug is considered to enable the continuous administration of the drug to the patient with the advanced symptom for a long period with avoiding the difficulty in taking it and to be particularly useful.

However, it is said that it is difficult to absorb a drug, of which the amount is effective for a sufficient effect, into the body through the skin since the skin generally has low drug permeability. To overcome such difficulty, a percutaneous absorption preparation comprising an anti-dementia drug has conventionally been studied.

For example, Japanese Patent Laid-Open No. 11-315016 (Patent Literature 1) discloses an ointment and the like for transdermal administration of an anti-dementia drug and a suppository for rectal administration, wherein the transdermal absorbability of donepezil hydrochloride is improved by a substrate comprising a higher alcohol and an ester derivative thereof.

In addition, WHO 03/032960 (Patent Literature 2) discloses a transdermal absorption-type anti-dementia preparation comprising an adhesion composition, wherein the adhesion composition contains a dispersed active ingredient, the active ingredient is released at a pharmacologically effective rate, and a skin permeation rate is at least 1.2 µg/cm2 or more per hour. Further, in the examples, disclosed is a preparation comprising an adhesion composition, prepared by containing donepezil hydrochloride which is an active ingredient, a styrene-isoprene-styrene block copolymer which is a hydrophobic polymer, and sodium acetate which is an organic acid salt, wherein the size of the preparation for single-dose administration for 24 hours is 60 cm2.

In addition, in a transdermal preparation, a drug which is its active ingredient needs to be retained without precipitating in the preparation and to be stably placed on the skin. Thus, in consideration of, e.g., improvement of these functions, percutaneous absorption preparations and materials thereof have been examined.

For example, Japanese Patent Laid-Open No. 10-182439 (Patent Literature 3) discloses an adhesion- and bonding agent for a skin or transdermal treatment system, the adhesion-bonding agent comprising a (meth)acrylate copolymer containing a tertiary or quaternary amino group, an acrylate- or (meth)acrylate polymer or copolymer containing an acidic group, and a softening agent. As such softening agents in the examples, triethyl citrate and acetyl triethyl citrate are disclosed.

In addition, WO 02/38139 (Patent Literature 4) discloses a percutaneous absorption preparation comprising a polymer compound having an amino group, a drug forming an acid addition salt, and carboxylic acid and/or a salt thereof.

In addition, Japanese Patent Laid-Open No. 4-117323 (Patent Literature 5) discloses a transdermally absorbable patch prepared by retaining a patch layer containing an adhesive base and a drug on a support, wherein the transdermally absorbable patch comprises a certain amount of the drug in acid addition form and a polymer that contains a certain amount of basic nitrogen and has no adhesiveness on the skin at room temperature.

In addition, WO 2007/129427 (Patent Literature 6) discloses a reservoir-type percutaneous absorption preparation comprising an anti-dementia drug, wherein the percutaneous absorption preparation comprises at least an adhesive layer, an interlayer, and a drug-containing layer in this order from the side to be applied to the skin. The drug-containing layer of the percutaneous absorption preparation comprises at least an anti-dementia drug, a polymer compound having an amino group, a polyhydric alcohol, and one or more carboxylate esters and a period of applying the preparation is reported to be able to be set to be a long period even in the case of single-dose administration.

However, in percutaneous absorption preparations, maintenance of safety for the skin of a patient is a precondition for treatment, and, in a percutaneous absorption preparation comprising an anti-dementia drug, it is also still demanded to reduce irritation to the skin of a patient.

### [Citation List]

### [Patent Literature]

[Patent Literature 1]
   Japanese Patent Laid-Open No. 11-315016
[Patent Literature 2]
   WO 03/032960
[Patent Literature 3]
   Japanese Patent Laid-Open No. 10-182439
[Patent Literature 4]
   WO 02/38139
[Patent Literature 5]
   Japanese Patent Laid-Open No. 4-117323
[Patent Literature 6]
   WO 2007/129427

### [SUMMARY OF INVENTION]

An object of the present invention is to provide a novel percutaneous absorption preparation that is significantly low skin irritation.

The percutaneous absorption preparation according to the present invention comprises a drug-containing layer comprising an anti-dementia drug, a polymer compound having an amino group, a polyhydric alcohol fatty acid ester, a polyhydric alcohol, a polyvalent carboxylate ester, and a styrenic polymer compound, wherein the content of the anti-dementia drug is 0.5-20 mass% of the drug-containing layer.

When the percutaneous absorption preparation according to the present invention is used by being applied to a patient once daily, skin irritation can significantly be suppressed.

### [BRIEF DESCRIPTION OF DRAWINGS]

[Figure 1] Figure 1 illustrates an embodiment of a percutaneous absorption preparation according to the present invention. A is a cross-sectional view of the percutaneous absorption preparation; and B is a skin contact surface view of the percutaneous absorption preparation.
[Figure 2] Figure 2 is a graph indicating the results of an *in vitro* human dermal penetration test using a transdermally absorbable agent according to the present invention.

### [DESCRIPTION OF EMBODIMENTS]

### Definition

As used herein, "alkyl" means straight-chain, branched, or cyclic alkyl, preferably having 2 to 18 carbon atoms in total.
In addition, as used herein, "alcohol" means a straight-chain, branched, or circular saturated or unsaturated alcohol.

### Percutaneous absorption preparation

The percutaneous absorption preparation according to the present invention has one feature of comprising the drug-containing layer having specific composition as described above. Such a percutaneous absorption preparation is particularly advantageous for effectively treating dementia while suppressing skin irritation when being applied once daily,

The content of the anti-dementia drug is preferably 0.5-20 mass%, more preferably 1-20 mass%, further preferably 3.0-15.0 mass%, further preferably 6.25-12.5 mass%, of the drug-containing layer. Setting of the content of the anti-dementia drug in such a manner is preferred for efficient transdermal administration of the drug while suppressing skin irritation.

In addition, the anti-dementia drug according to the present invention is preferably a basic drug. In addition. In accordance with a preferred embodiment of the present invention, the anti-dementia drug is a nitrogen-containing basic drug or a salt thereof and such salts include, for example, hydrochlorides, tartrates, and hydrobromates, but are not limited thereto if being pharmaceutically acceptable salts.
Further, the above-described basic drug or the salt thereof is preferable donepezil hydrochloride, memantine hydrochloride, rivastigmine tartrate, galantamine hydrobromide, or tacrine hydrochloride, more preferably donepezil hydrochloride.

In addition, the polymer compound having an amino group in the drug-containing layer is preferably a copolymer composed of a dialkylaminoalkyl (meth)acrylate and a monomer unit selected from an alkyl (meth)acrylate, a hydroxyalkyl (meth)acrylate, and a combination thereof.
Such a copolymer is advantageous for stably retaining a drug and achieving a good drug flux.

Further, the polymer compound having an amino group is preferably an acryl (meth)acrylate-alkyl (meth)acrylate-dialkylaminoethyl (meth)acrylate copolymer, more preferably a copolymer comprising di-C1-2-alkylamino-C1-2-alkyl (meth)acrylate and C1-4 alkyl (meth)acrylate and monohydroxy-C2-4-alkyl (meth)acrylate as monomer units, further preferably a methyl (meth)acrylate-butyl (meth)acrylate-dimethylaminoethyl (meth)acrylate copolymer, further preferably a methyl methacrylate-butyl methacrylate-dimethylaminoethyl methacrylate copolymer. Such a methyl methacrylate-butyl methacrylate-dimethylaminoethyl methacrylate copolymer is commercially available, for example, as Eudragit® E100 (Degussa AG).

In addition, in the above-described polymer compound having an amino group, the molar ratio, the molecular weight, and the like of a monomer unit may appropriately be regulated by the person skilled in the art.

In addition, the content of the polymer compound having an amino group in the drug-containing layer is, without particular limitation, preferably 5-30 mass%, more preferably 10-25 mass%.

The above-described polyvalent carboxylate ester is preferably a divalent to hexavalent carboxylate ester, a divalent to trivalent C1-6 alkyl carboxylate ester, more preferably a divalent to trivalent C1-6 alkyl carboxylate ester, further preferably a divalent to trivalent C1-3 alkyl carboxylate ester, further preferably a tri-C1-3-alkyl citrate ester or a di-C1-3-alkyl sebacate ester, further preferably triethyl citrate or diethyl sebacate.

In addition, the content of the polyvalent carboxylate ester in the drug-containing layer is preferably 1-10 mass%, more preferably 2-5 mass%.

In addition, the polyhydric alcohol is preferably a sugar-alcohol or a glycol, more preferably a glycerol or a glycol, further preferably at least one selected from the group consisting of trotyls, pentitols, hexitols, and glycols. More specifically, the polyhydric alcohol is selected from glycerols, propylene glycol, dipropylene glycol, butylene glycol, d-sorbitol, xylitol, mannitol, polyethylene glycol, and combinations thereof, more preferably a glycerol.

In addition, the content of the polyhydric alcohol in the drug-containing layer is preferably 1-10 mass%, more preferably 3-10 mass%.

The polyhydric alcohol fatty acid ester is preferably a sugar-alcohol fatty acid ester or a glycol fatty acid ester, more preferably a sugar-alcohol fatty acid ester, further preferably at least one selected from the group consisting of sorbitan fatty acid ester, propylene glycol fatty acid ester, and glycerol fatty acid ester, further preferably sorbitan fatty acid ester.
As more specific examples, the polyhydric alcohol fatty acid esters include sorbitan monolaurate, sorbitan monostearate, sorbitan monooleate, sorbitan monopalmitate, sorbitan trioleate, or sorbitan tristearate, preferably sorbitan monolaurate.

In addition, the content of the polyhydric alcohol fatty acid ester in the drug-containing layer is preferably 3-15 mass%, more preferably 3-10 mass%.

In addition, the styrenic polymer compound is, without particular limitation unless precluding the release and retention of the drug, preferably a copolymer of styrene with a polymerizable alkene having 2 to 8 carbon atoms, more preferably a styrene-isoprene-styrene block copolymer, a styrene-butylene-styrene block copolymer, or a styrene-butadiene-styrene block copolymer, further preferably a styrene-isoprene-styrene block copolymer.

In addition, in the styrenic polymer compound, the molar ratio, the molecular weight, and the like of a monomer unit is appropriately regulated by the person skilled in the art.

In addition, the content of the styrenic polymer compound in the drug-containing layer is preferably 5-80 mass%, more preferably 15-70 mass%.

### Combination

The drug-containing layer according to the present invention may be formed by appropriately combining such components as described above as far as the components and the amounts thereof are used.
In accordance with a preferred embodiment of the present invention, the drug-containing layer comprises a basic anti-dementia drug or a salt thereof; a polymer compound having an amino group, which is a copolymer composed of a dialkylaminoalkyl (meth)acrylate and a monomer unit selected from an alkyl (meth)acrylate, a hydroxyalkyl (meth)acrylate, and a combination thereof; a polyhydric alcohol fatty acid ester; a polyhydric alcohol; a polyvalent carboxylate ester; and a styrenic polymer compound.

In addition, in accordance with a further preferred embodiment of the present invention, the drug-containing layer comprises a basic anti-dementia drug or a salt thereof, a polymer compound having an amino group, which is a copolymer composed of a dialkylaminoalkyl (meth)acrylate and a monomer unit selected from an alkyl (meth)acrylate, a hydroxyalkyl (meth)acrylate, and a combination thereof; a polyhydric alcohol fatty acid ester; a polyhydric alcohol; a polyvalent carboxylate ester; and a styrenic polymer compound which is a copolymer of styrene with a polymerizable alkene having 2 to 8 carbon atoms.

In addition, in accordance with a further preferred embodiment of the present invention, the drug-containing layer comprises a basic anti-dementia drug or a salt thereof, a polymer compound having an amino group, which is a copolymer composed of a dialkylaminoalkyl (meth)acrylate and a monomer unit selected from an alkyl (meth)acrylate, a hydroxyalkyl (meth)acrylate, and a combination thereof; a polyhydric alcohol fatty acid ester; a polyhydric alcohol; a polyvalent carboxylate ester which is a divalent to hexavalent carboxylate ester; and a styrenic polymer compound which is a copolymer of styrene with a polymerizable alkene having 2 to 8 carbon atoms.

In addition, in accordance with a further preferred embodiment of the present invention, the drug-containing layer comprises a basic anti-dementia drug or a salt thereof, a polymer compound having an amino group, which is a copolymer composed of a dialkylaminoalkyl (meth)acrylate and a monomer unit selected from an alkyl (meth)acrylate, a hydroxyalkyl (meth)acrylate, and a combination thereof; a polyhydric alcohol fatty acid ester; a polyhydric alcohol which is a sugar-alcohol or a glycol; a polyvalent carboxylate ester which is a divalent to hexavalent carboxylate ester; and a styrenic polymer compound which is a copolymer of styrene with a polymerizable alkene having 2 to 8 carbon atoms.

In addition, in accordance with a further preferred embodiment of the present invention, the drug-containing layer comprises a basic anti-dementia drug or a salt thereof, a polymer compound having an amino group, which is a copolymer composed of a dialkylaminoalkyl (meth)acrylate and a monomer unit selected from an alkyl (meth)acrylate, a hydroxyalkyl (meth)acrylate, and a combination thereof; a polyhydric alcohol fatty acid ester which is a sugar-alcohol fatty acid ester or a glycol fatty acid ester; a polyhydric alcohol which is a sugar-alcohol or a glycol; a polyvalent carboxylate ester which is a divalent to hexavalent carboxylate ester; and a styrenic polymer compound which is a copolymer of styrene with a polymerizable alkene having 2 to 8 carbon atoms.

In addition, in accordance with a further preferred embodiment of the present invention, the drug-containing layer comprises a basic anti-dementia drug or a salt thereof; a polymer compound having an amino group, which is a methyl methacrylate-butyl methacrylate-dimethylaminoethyl methacrylate copolymer; a polyhydric alcohol fatty acid ester which is a sugar-alcohol fatty acid ester; a polyhydric alcohol which is a glycerol or a glycol; a polyvalent carboxylate ester which is a sebacate ester or a citrate ester; and a styrenic polymer compound which is a styrene-isoprene-styrene block copolymer, a styrene-butylene-styrene block copolymer, or a styrene-butadiene-styrene block copolymer.

In addition, in any of the above-described preferred combinations, the basic anti-dementia drug or the salt thereof is preferably donepezil hydrochloride, memantine hydrochloride, rivastigmine tartrate, galantamine hydrobromide, or tacrine hydrochloride, more preferably donepezil hydrochloride.
In addition, in any of the above-described preferred combinations, the polymer compound having an amino group is preferably an acryl (meth)acrylate-alkyl (meth)acrylate-dialkylaminoethyl (meth)acrylate copolymer, more preferably a methyl methacrylate-butyl methacrylate-dimethylaminoethyl methacrylate copolymer.
In addition, in any of the above-described preferred combinations, the polyhydric alcohol fatty acid ester is preferably a sugar-alcohol fatty acid ester, more preferably a sorbitan fatty acid ester.

In addition, in any of the above-described preferred combinations, the polyhydric alcohol is preferably a glycerol or a glycol, more preferably a glycerol.
In addition, in any of the above-described preferred combinations, the polyvalent carboxylate ester is preferably a divalent to hexavalent carboxylate ester, more preferably a divalent to trivalent C₁₋₆ alkyl carboxylate ester, further preferably a sebacate ester or a citrate ester.
In addition, in any of the above-described preferred combinations, the styrenic polymer compound is preferably a styrene-isoprene-styrene block copolymer, a styrene-butylene-styrene block copolymer, or a styrene-butadiene-styrene block copolymer, more preferably a styrene-isoprene-styrene block copolymer.

In addition, the thickness of the drug-containing layer according to the present invention, which thickness is appropriately determined in consideration of a drug amount and/or the like by the person skilled in the art, may be, for example, 30-150 µm.

### Fixing means

The drug-containing layer of the percutaneous absorption preparation according to the present invention may also be utilized as a percutaneous absorption preparation without being processed and preferably comprises a fixing means for the drug content layer.

A preferred embodiment of the percutaneous absorption preparation according to the present invention is described below with reference to schematic views.

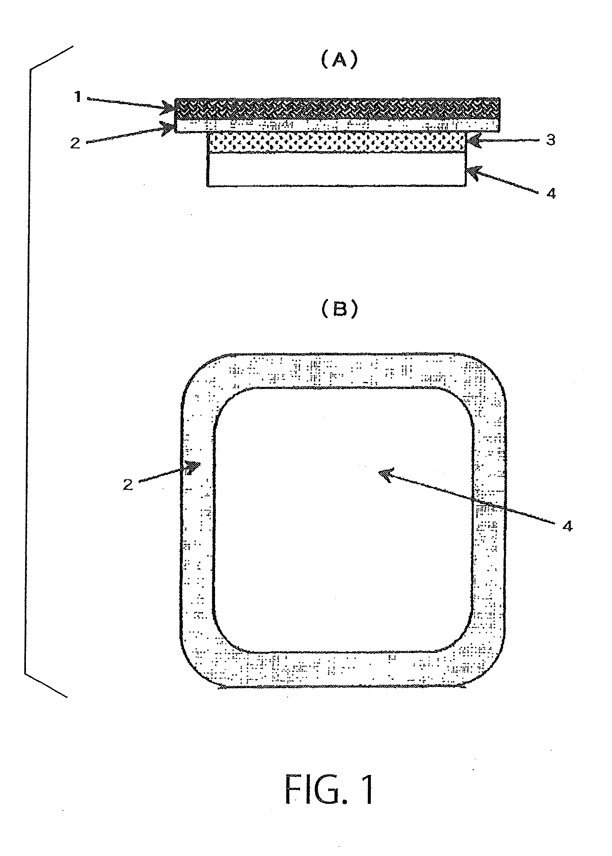

Figure 1A is a cross-sectional view illustrating one embodiment of the percutaneous absorption preparation according to the present invention.
As illustrated in Figure 1A, the percutaneous absorption preparation comprises: a layered product comprising a drug-containing layer 4 and a support layer 3 sequentially from the skin side; and a fixing means (1, 2) which can fix the layered product on the skin 2.

In addition, the fixing means (1, 2) comprises a cover layer 1 which coats the layered product; and an adhesive layer 2 which adheres the cover layer 1 to the skin. The cover layer 1 is constituted to be able to coat the part other than the skin contact surface of the layered product. In addition, the adhesive layer 2 is placed on the side, closer to the skin, of the cover layer 1. In addition, Figure 1B illustrates the skin contact surface of the percutaneous absorption preparation, in which the adhesive layer 2 is placed in the periphery/terminus of the drug-containing layer 4 and can fix the percutaneous absorption preparation on the skin by adhering to the skin. Such a constitution is advantageous for maintaining the adhesion stability of the percutaneous absorption preparation to the skin.
The drug-containing layer may contact with the skin without being processed or a drug-permeable polymer membrane for regulating drug permeability may be disposed on a part of one side, closer to the skin, of the drug-containing layer, and the present invention also encompasses such embodiments.

In addition, the adhesive layer is, without particular limitation if being a biocompatible material which enables adhesion of the percutaneous absorption preparation to the skin, preferably polyacrylate, polydimethylsiloxane, polyisobutylene, combinations thereof, or the like. Further, for example, a known tackifier and/or the like may also appropriately be added to the constituent material of the adhesive layer. The above-mentioned material may also be used as an auxiliary adhesive agent to be added to the surface of the drug-permeable membrane.
In addition, the contact area of the adhesive layer to the skin can appropriately be determined in consideration of the area of the drug-permeable membrane, an administration period and/or the like, an application site, and/or the like.

In addition, the drug-permeable polymer membrane is preferably a microporous membrane having pores permeable to a drug but is not limited thereto as far as release of the drug to the skin can be controlled. The constituent materials of the drug-permeable polymer membrane include, without particularly rlimitation, EVA (ethylene-vinyl acetate copolymers), polyethylene, polypropylene, polyacrylonitrile, polymethyl methacrylate, combinations thereof, and the like.

The support may be elastic or non-elastic. Specific materials constituting the support include, without particular limitation as far as the agent-containing layer can be isolated from another member, for example, woven fabrics, non-woven fabrics, PET (polyethylene terephthalate), polyurethane, polyester, polyethylene, composite materials thereof, or the like.
In addition, the same materials as those of the support may be used for the cover.

### Production Method

A preferred method for producing the percutaneous absorption preparation according to the present invention is as follows.
First, the components of the drug-containing layer according to the present invention are appropriately mixed in a solvent to adjust a liquid mixture containing the components in the solvent. Then, the liquid mixture is used as a plaster solution and applied onto a liner. Then, the plaster solution is dried at around 60-120°C to obtain the drug-containing layer and, as needed, a support is laminated thereon to obtain a layered product. Then, a cover, on one surface of which an adhesive layer is placed, is prepared. Then, the one surface, which is closer to the adhesive layer, of the cover and one surface, which is closer to the support, of the layered product are affixed to each other to obtain a percutaneous absorption preparation. In this case, the sizes of the adhesive layer and the cover are pre-adjusted so that the adhesive layer coats the periphery or the terminus of the one side, closer to the skin, of a drug-permeable membrane.

In the above-described production method, examples of the solvent which is used when the drug-containing layer and the adhesive layer are prepared include ethyl acetate, butyl acetate, toluene, n-hexane, n-heptane, tetrahydrofuran, dimethylformamide, methanol, ethanol, or the like.

In addition, the percutaneous absorption preparation according to the present invention is preferably applied around once daily in consideration of the suppression of skin irritation and the stable and efficient administration of a drug. Accordingly, in accordance with another embodiment of the invention, provided is a method for treating dementia, comprising applying the above-described percutaneous absorption preparation to the skin of an organism once daily.

In addition, such organisms as described above include rabbit, dog, human, or the like, preferably human.

### [Examples]

The present invention will be specifically described below with reference to Examples but is not limited to these Examples.

### Example 1: Drug-containing Layer Containing 12.5 mass% of Donepezil Hydrochloride

### Preparation of Drug-containing Layer

Donepezil hydrochloride, Eudragit^{®} E100, triethyl citrate, glycerol, and SML (sorbitan monolaurate) were prepared in the the-described formulation ratio and mixed and stirred in an appropriate amount of toluene. SIS (styrene-isoprene-styrene block copolymer, Kraton^{®} D1111K, manufactured by Kraton Corporation) was added in a formulation ratio as described below to the obtained liquid mixture to obtain a plaster solution.

**[Table 1]**

| Constituent | mass% |
|---|---|
| Donepezil hydrochloride | 12.5 |
| Eudragit^{®} E100 | 17.67 |
| Triethyl citrate | 10 |
| Glycerol | 10 |
| SML | 5 |
| SIS | 44.83 |

The above-described ointment solution was applied onto a liner made of polyethylene terephthalate and dried at 80°C for 15 minutes to obtain a drug-containing layer. The amount of the drug-containing layer after the drying was adjusted to be 50 g/m².
Then, a support layer (Scotchpak™ 9732, manufactured by 3M Company) was laminated on the opposite side of the liner of the drug-containing layer. Then, the liner is peeled from the drug-containing layer to obtain the layered product.

### Disposition of Fixing means

Duro-Tak^{™} 87-2287 (manufactured by National Starch & Chemical) was applied onto a liner made of polyethylene terephthalate and dried at 80°C for 15 minutes to obtain an adhesive layer. The weight of the adhesive layer after the drying was 100 g/m². Then, a cover layer (polyester woven fabric) was laminated on the opposite side of the adhesive layer to the liner to obtain a fixing means.
Then, the liner on the adhesive layer of the fixing means was peeled and the support layer of the pre-cut layered product and the adhesive layer of the fixing means were affixed to each other. Then, a liner made of polyethylene terephthalate was affixed on a surface formed with the adhesive layer and the drug-containing layer, a skin contact surface was prepared, and cutting was performed to obtain a percutaneous absorption preparation having the same constitution as in Figure 1.

### Example 2: Drug-containing Layer Containing 6.25 mass% of Donepezil Hydrochloride

A percutaneous absorption preparation was prepared by the same procedure as in Example 1 except that the amount of donepezil hydrochloride in the drug-containing layer was changed to 6.25 mass% in Example 1. The amounts of the other constituents than donepezil hydrochloride were not changed from those of Example 1 and the percentages of the respective constituents based on the drug-containing layer are listed as the following formulation.

**[Table 2]**

| Constituent | mass% |
|---|---|
| Donepezil hydrochloride | 6.25 |
| Eudragit^{®} E100 | 17.67 |
| Triethyl citrate | 10 |
| Glycerol | 10 |
| SML | 5 |
| SIS | 51.08 |

### Comparative Example 1: Drug-containing Layer Containing 25 mass% of Donepezil Hydrochloride

A percutaneous absorption preparation was prepared by the same procedure as in Example 1 except that the amount of donepezil hydrochloride in the drug-containing layer was changed to twice that in Example 1. The amounts of the other constituents than donepezil hydrochloride were not changed from those of Example 1 and the percentages of the respective constituents based on the drug-containing layer are listed as the following formulation.

**[Table 3]**

| Constituent | mass% |
|---|---|
| Donepezil hydrochloride | 25 |
| Eudragit^{®} E100 | 17.67 |
| Triethyl citrate | 10 |
| Glycerol | 10 |
| SML | 5 |
| SIS | 32.33 |

### Test Example 1

The percutaneous absorption preparations (20 mm in diameter) were applied to the backs of rabbits (Japanese white species, male, 16-23-week-old, n = 6) for 24 hours. Skin irritation indices for erythema and edema were calculated with reference to Draize skin irritation evaluation criteria and a evaluation procedure therefor 1, 24, and 48 h after removal of the preparations.

**[Table 4]**

| Draize Skin Irritation evaluation Criteria | |
|---|---|
| Evaluation Criteria for Skin Reaction | Score |
| Erythema and eschar formation | |
| No erythema | 0 |
| Very slight erythema (barely perceptible) | 1 |
| Well defined erythema | 2 |
| Moderate to severe erythema | 3 |
| Severe erythema (beet redness) to slight eschar formation (injuries in depth) | 4 |

| Edema | |
|---|---|
| No edema | 0 |
| Very slight edema (barely perceptible) | 1 |
| Slight edema (edges of area well defined by definite raising) | 2 |
| Moderate edema (raised approximately 1 mm) | 3 |
| Severe edema (raised more than 1 mm and extending beyond area of exposure) | 4 |

The results are listed in Table 5.

**[Table 5]**

| Preparation | Skin irritation index* |
|---|---|
| Example 1 | 1.06 |
| Example 2 | 0.56 |
| Comparative Example 1 | 1.72 |

| | |
|---|---|
| *Abraded skin was not evaluated. | |

(Skin irritation index in Example 1) / (skin irritation index in Comparative Example 1) was 0.61; and (skin irritation index in Example 2) / (skin irritation index in Comparative Example 1) was 0.33.
The skin irritations in Examples 1 and 2 were confirmed to be lower than that in Comparative Example 1.

### Test Example 2

### In Vitro Hairless Mouse Dermal Penetration Test (24 hours)

The percutaneous absorption preparation (application area of 2.5 cm2) of Example 1 or Comparative Example 1 was applied to the stratum corneum layer side of the hairless mouse skin and set in a flow-through cell (5 cm2) through which warm water was circulated so that a skin surface was at about 32°C. A phosphate buffered saline solution (pH 7.4) was used as a receiver phase and the receiver pahse was sampled at a rate of 2.5 mL/hr every 4 hours until 24 hours. For the sampled solution, a drug amount was measured by HPLC, a penetration rate per hour was calculated, and the mean value of a flux (mcg/cm2/hr) per unit area was determined every 4 hours.

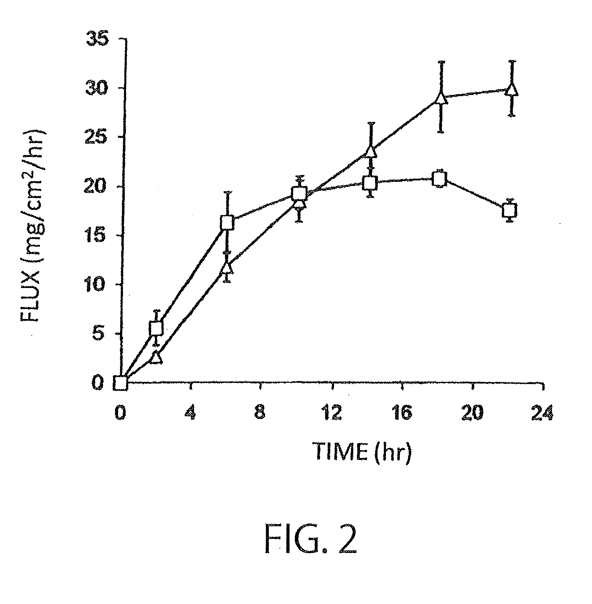

The results are as indicated in Figure 2. Example 1 is represented by squares; and Comparative Example 1 is represented by triangles.

In addition, as a result of calculating dermal penetration amounts from the sampled solutions, the dermal penetration amount of Example 1 was 399.92 ± 26.90 (mcg/cm²) and the dermal penetration amount of Comparative Example 1 was 463.23 ± 52.02 (mcg/cm²).

In addition, as a result of calculating the residual drug amount rates of Example 1 and Comparative Example 1 in the preparations based on the dermal penetration amounts, the residual drug amount rate of Example 1 (12.5 mass% drug) was 29.4-34.5% and the rate of Comparative Example 1 (25 mass% drug) was 54.6-67.2%.
The drug was confirmed to be more efficiently absorbed in Example 1 than Comparative Example 1.

### Test Example 3

As a result of conducting the same test as in Test Example 2 using Example 2 (6.25 mass% drug), the residual drug amount rate of Example 2 was 35.1-46.2%.
The drug was confirmed to be more efficiently absorbed in Example 2 than Comparative Example 1 in Test Example 2.

## Claims

1. A percutaneous absorption preparation comprising a drug-containing layer comprising an anti-dementia drug, a polymer compound having an amino group, a polyhydric alcohol fatty acid ester, a polyhydric alcohol, a polyvalent carboxylate ester, and a styrenic polymer compound,
wherein the content of the anti-dementia drug is 0.5-20 mass% of the drug-containing layer.

2. The percutaneous absorption preparation according to claim 1, for being applied to human once daily.

3. The percutaneous absorption preparation according to claim 1 or 2, wherein the anti-dementia drug is a basic drug or a salt thereof.

4. The percutaneous absorption preparation according to any one of claims 1 to 3, wherein the anti-dementia drug is donepezil hydrochloride, memantine hydrochloride, rivastigmine tartrate, galantamine hydrobromide, or tacrine hydrochloride.

5. The percutaneous absorption preparation according to any one of claims 1 to 4, wherein the polymer compound having an amino group is a copolymer composed of a dialkylaminoalkyl (meth)acrylate and a monomer unit selected from an alkyl (meth)acrylate, a hydroxyalkyl (meth)acrylate, and a combination thereof.

6. The percutaneous absorption preparation according to any one of claims 1 to 5, wherein the polymer compound having an amino group is an acryl (meth)acrylate-alkyl (meth)acrylate-dialkylaminoethyl (meth)acrylate copolymer.

7. The percutaneous absorption preparation according to any one of claims 1 to 5, wherein the polyvalent carboxylate ester is a divalent to hexavalent carboxylate ester.

8. The percutaneous absorption preparation according to any one of claims 1 to 7, wherein the polyvalent carboxylate ester is a sebacate ester or a citrate ester.

9. The percutaneous absorption preparation according to any one of claims 1 to 8, wherein the polyhydric alcohol is a sugar-alcohol or a glycol.

10. The percutaneous absorption preparation according to any one of claims 1 to 9, wherein the polyhydric alcohol is a glycerol or a glycol.

11. The percutaneous absorption preparation according to any one of claims 1 to 10, wherein the polyhydric alcohol fatty acid ester is a sugar-alcohol fatty acid ester or a glycol fatty acid ester.

12. The percutaneous absorption preparation according to any one of claims 1 to 11, wherein the polyhydric alcohol fatty acid ester is a sorbitan fatty acid ester.

13. The percutaneous absorption preparation according to any one of claims 1 to 12, wherein the styrenic polymer compound is a copolymer of styrene with a polymerizable alkene having 2 to 8 carbon atoms.

14. The percutaneous absorption preparation according to any one of claims 1 to 13, wherein the styrenic polymer compound is a styrene-isoprene-styrene block copolymer, a styrene-butylene-styrene block copolymer, or a styrene-butadiene-styrene block copolymer.

15. The percutaneous absorption preparation according to any one of claims 1 to 14, further comprising:
a layered product comprising the drug-containing layer and a support layer sequentially from the skin side; and
a fixing means which can fix the layered product on a skin.
